# FASCICULE DE BREVET EUROPEEN

(11) **EP 2 809 282 B1**
(45) Date de publication et mention de la délivrance du brevet: **06.04.2016**
(21) Numéro de dépôt: 13712335.2
(22) Date de dépôt: 29.01.2013
(51) Int. Cl.: A61F 13/02, A61F 13/42, A61F 13/00

(54) **PANSEMENT MUNI D'UN SYSTEME DE DETECTION**
WUNDVERBAND MIT DETEKTIONSSYSTEM
WOUND DRESSING PROVIDED WITH A DETECTION SYSTEM

(30) Priorité: 30.01.2012 FR 1250847
(43) Date de publication de la demande: 10.12.2014
(73) Titulaire: Commissariat à l'Énergie Atomique et aux Énergies Alternatives, 75015 Paris (FR)
(72) Inventeur: MARSIQUET, Cyril, F-16270 Roumazieres Laubert (FR); REVOL-CAVALIER, Frédéric, F-38180 Seyssins (FR)
(74) Mandataire: Nony
(86) Numéro de dépôt international: PCT/IB2013/050747
(87) Numéro de publication internationale: WO 2013/114273

(56) Documents cités:
- WO-A1-2011/004165
- WO-A1-2012/012286
- DE-A1- 4 014 572
- US-A- 4 382 441

## Description

La présente invention concerne les pansements et systèmes utilisés pour améliorer le confort des patients sur lesquels ces pansements sont appliqués.

En Europe, le nombre de plaies chroniques est en constante augmentation et cela nécessite dans la plupart des cas une hospitalisation. Cette dernière s'accompagne de coûts de santé et de soins médicaux très élevés.

A l'heure actuelle, un pansement est changé par le personnel médical à intervalles réguliers et indépendamment de son taux de remplissage.

Ainsi, dans certains cas, le pansement est changé alors qu'il aurait pu être laissé en place, ce qui génère des coûts de personnel inutiles.

Une approche permettant de répondre à ce problème consiste à instrumenter le pansement afin de suivre en temps réel non seulement l'évolution de la cicatrisation mais également celle du pansement lui-même.

Instrumenter un pansement avec un capteur de remplissage permet d'optimiser le moment du changement du pansement et d'aider le personnel médical à organiser son temps.

L'article David McColl, Brian Cartlidge, Patricia Connolly, Real-time monitoring of moisture levels in wound dressings *in vitro*: An experimental study. International Journal of Surgery (2007) 5, 316e322 relate une méthode de mesure capable de suivre l'évolution du taux d'humidité dans un pansement en temps réel.

Le principe utilisé est la mesure d'impédance électrique, permettant la détection de liquides ioniques. Plus le volume de liquide est élevé dans le pansement, plus l'impédance est faible. Plusieurs couples d'électrodes sont répartis à l'interface plaie/pansement et permettent ainsi d'avoir une cartographie du taux d'humidité du pansement.

Cette mesure comporte cependant un inconvénient, à savoir qu'elle a lieu uniquement à l'interface plaie/pansement. De ce fait, le taux d'humidité mesuré peut ne pas correspondre à celui de la partie absorbante du pansement.

La publication WO 99/17692 présente un capteur de saturation avec indicateur destiné aux tampons absorbants pour l'hygiène féminine. Ce capteur de saturation est formé par plusieurs détecteurs d'humidité répartis dans la longueur du tampon. Chaque détecteur est composé de deux lamelles et d'un absorbant placé entre les deux. Le détecteur devient conducteur lorsque l'absorbant est mouillé. La mesure du taux d'humidité étant ponctuelle, ce système de détection suppose que la migration du fluide corporel se fait suivant l'axe longitudinal du tampon et de manière homogène.

L'invention vise à perfectionner encore les pansements en permettant de connaître leur taux de remplissage.

L'invention a ainsi pour objet un pansement comportant :
- une face d'application sur une plaie ;
- une structure absorbante pour absorber un liquide émis provenant de la plaie ;
- une structure intermédiaire située entre la face d'application et la structure absorbante et agencée pour privilégier la diffusion du liquide depuis la face d'application vers une ou plusieurs zones d'admission, d'étendue restreinte, de la structure absorbante ; et
- un système de détection sensible à l'étendue de la structure absorbante mouillée par le liquide ayant pénétré dans celle-ci par la ou les zones d'admission précitées.

Par zone « d'étendue restreinte », il faut comprendre que l'étendue de la zone est inférieure à celle de la structure absorbante.

Contrairement à la mesure du taux d'humidité faite par McColl où les électrodes ne font pas partie de la structure absorbante et à la publication WO 99/17692 qui ne mesure le taux d'humidité qu'à des endroits précis du tampon, l'invention permet de détecter le niveau de remplissage de la structure absorbante de manière surfacique et de connaître l'évolution de la surface humide dans le temps. La surface mesurée comme humide correspond à la surface réelle de la structure absorbante qui est humide.

La structure intermédiaire comporte de préférence un film formant barrière au liquide, traversé par une ou plusieurs ouvertures en correspondance avec la ou lesdites zones d'admission, notamment entre 1 et 10 ouvertures. En variante, ou additionnellement, la structure intermédiaire privilégie la diffusion du liquide vers une ou plusieurs zones d'admission grâce à une anisotropie du ou des matériaux qui la constitue ou à un traitement, par exemple d'imprégnation, qui obture les pores sur la face de la structure intermédiaire en regard de la structure absorbante. On peut encore obstruer les pores de la structure absorbante sur sa face tournée vers la plaie, à l'exception de la ou des zones d'admission.

La ou l'ensemble desdites zones d'admission correspondent de préférence à moins de 20 % de la surface totale de la structure absorbante, en particulier moins de 5 % de la surface totale de la structure absorbante.

Le film formant barrière, lorsque présent, est de préférence hydrophobe. Le film formant barrière est de préférence en un matériau semi-perméable, étant par exemple en polyuréthane. Par semi-perméable, on entend un matériau perméable à un gaz, tel que l'air et imperméable à un liquide. Ainsi, le pansement conserve sa propriété respirante.

Le système de détection comporte de préférence au moins un réseau d'électrodes, notamment deux réseaux d'électrodes agencés sous forme de grille. Le ou les réseaux d'électrodes sont isolés électriquement entre eux, lorsque les électrodes se croisent. Lorsque le système de détection comporte deux réseaux d'électrodes agencés sous forme de grille, des matrices ligne et colonne peuvent être construites avec les valeurs des mesures entre les couples d'électrodes consécutives de chaque réseau, et un produit matriciel des matrices ligne et colonne effectué pour aboutir à une matrice résultat dont les coefficients sont représentatifs du degré d'humidité en divers emplacements de la structure absorbante dont les coordonnées sont associées aux coefficients des matrices ligne et colonne.

Il est alors possible de générer une information signalant non seulement la préférence d'humidité ou non à tel emplacement mais également de quantifier le degré d'humidité de chaque emplacement, ce qui peut servir de base à une représentation cartographique où les degrés d'humidité sont signalés par des couleurs différentes.

Les électrodes peuvent être portées par un support ne gênant pas la diffusion du liquide et non occlusif. Ce support peut être figuré, par exemple, par un film hydrophobe, tel que par exemple un film non tissé hydrophobe. Cela permet d'éviter que le liquide ne s'étale entre les différentes électrodes.

Le support des électrodes peut, le cas échéant, être le film formant barrière ou la structure absorbante elle-même, ou une enveloppe externe du pansement.

L'agencement des électrodes peut être tel qu'il ne nécessite pas de disposer les électrodes sur un support, ce qui est le cas par exemple lorsque les électrodes sont disposées selon un grille.

Le système de détection comporte de préférence plusieurs emplacements de mesure répartis sur la structure absorbante, de façon à obtenir de préférence entre 2 et 100 emplacements de mesure, en particulier de 10 à 50 emplacements de mesure et plus particulièrement de 30 à 50 emplacements de mesure. Un emplacement de mesure peut être localisé entre deux électrodes consécutives du réseau d'électrodes. La mesure peut être effectuée sur sensiblement toute la longueur de ces électrodes au contact de la structure absorbante.

Les emplacements de mesure peuvent être répartis de façon régulière ou non sur la structure absorbante. Il peut s'avérer préférable de disposer d'emplacements de mesure plus resserrés lorsque l'on est loin de la ou des zones d'admission afin de bénéficier d'une résolution spatiale meilleure loin de la ou des zones d'admission, et de gagner ainsi en précision. Par exemple, l'écart entre les électrodes du réseau est plus faible à distance de la ou des zones d'admission qu'à proximité. Les électrodes peuvent être rectilignes ou non. Par exemple, dans le cas d'un agencement sous forme de grille, au moins certaines électrodes peuvent être curvilignes et contourner la ou les zones d'admission.

Les électrodes peuvent être filaires ou réalisées par sérigraphie, gravure ou métallisation. Lorsque les électrodes forment une grille, elles peuvent être reliées les unes aux autres à leurs intersections par un isolant électrique qui assure leur fixation.

Le système de détection peut être relié à un système de mesure qui détermine par exemple l'impédance entre deux électrodes consécutives, pour au moins plusieurs des couples d'électrodes consécutives pouvant être générés. Par exemple, le système de mesure injecte une tension alternative entre deux électrodes consécutives et mesure l'amplitude du courant circulant dans ces électrodes, ce courant étant par exemple déterminé par mesure de la tension de crête aux bornes d'une résistance parcourue par ce courant. Le système de détection peut être relié au système de mesure par une ou plusieurs connexions, de telle sorte que le système de mesure est aisément déconnectable du système de détection.

La tension alternative est par exemple un signal basse fréquence, de fréquence inférieure à 25 kHz. Le signal est par exemple de forme d'onde en créneaux. L'amplitude de la tension injectée est par exemple inférieure ou égale à 5 V.

Le système de détection couvre de préférence une surface de détection qui correspond à au moins 50 % de la surface totale de la structure absorbante.

Le système de détection peut être au contact d'une face de la structure absorbante opposée à la structure intermédiaire. En variante, le système de détection est au contact d'une face de la structure absorbante du côté de la structure intermédiaire. Ces deux variantes peuvent être combinées, le système de détection étant alors situé de part et d'autre de la structure absorbante.

La ou les zones d'admission précitées occupent de préférence une position centrale relativement à la structure absorbante. En variante, la ou les zones d'admission occupent une position excentrée relativement à la structure absorbante. Par exemple, le pansement comporte une zone unique d'arrivée du liquide dans la structure absorbante, située à proximité de sa périphérie, par exemple dans un coin pour un pansement de contour polygonal, notamment carré, ou en variante autant de zones d'admission qu'il y a de coins, disposées chacune dans un coin.

La structure absorbante peut être formée en tout ou partie d'un matériau alvéolaire, notamment à base de mousse de polyuréthane à cellules ouvertes et/ou d'un matériau fibreux, par exemple à base de fibres de cellulose.

La structure absorbante peut comporter un ou plusieurs agents superabsorbants, par exemple des polacrylates.

Le pansement selon l'invention peut comporter, entre la face d'application sur la plaie et la structure absorbante, une structure drainant le liquide émis provenant de la plaie.

La structure drainante peut comporter, voire être formée de, deux couches superposées, à savoir une couche proximale du côté de la plaie, drainant axialement le liquide, et une couche distale qui lui est superposée, drainant transversalement le liquide.

La couche proximale drainant axialement le liquide peut être formée en tout ou partie d'un matériau alvéolaire, tel qu'une mousse de polyuréthane à cellules ouvertes par exemple.

La couche distale drainant transversalement le liquide peut être formée en tout ou partie d'un matériau non tissé ou d'un tricot, notamment de viscose.

L'invention a encore pour objet un procédé d'estimation du degré de remplissage de la structure absorbante d'un pansement selon l'invention, tel que défini ci-dessus, dans lequel (i) on détecte grâce au système de détection l'étendue de la structure absorbante mouillée par le liquide provenant de la plaie ; et (ii) l'on détermine en fonction de cette étendue le degré de remplissage de la structure absorbante.

Le procédé peut comporter l'affichage d'une grandeur représentative du taux de remplissage, et/ou le déclenchement d'une alarme lorsque le taux de remplissage atteint une limite prédéfinie.

Le taux de remplissage peut correspondre au rapport de l'étendue détectée comme humide à l'étendue totale de la structure absorbante ; dans ce cas, un emplacement est considéré comme humide si la mesure à cet emplacement donne une mesure qui dépasse un seuil prédéfini.

En variante, le taux de remplissage repose sur une mesure quantitative en chaque emplacement du degré d'humidité et le taux de remplissage est calculé à partir des valeurs mesurées à chaque emplacement, par exemple des tensions de crête mesurées aux bornes de la résistance précitée.

Le procédé peut comporter l'étape consistant à mesurer une grandeur représentative de l'impédance électrique entre deux électrodes du système de détection, à l'aide d'un dispositif de mesure relié à celui-ci, notamment à deux électrodes consécutives.

L'étape (ii) peut être répétée pour mesurer successivement ladite impédance électrique entre différentes paires d'électrodes successives du ou de chaque réseau d'électrodes.

Le système de mesure peut être externe au pansement et les connexions avec le système de détection peuvent être établies avant un éventuel changement de pansement. En variante, le système de mesure est intégré au pansement, grâce par exemple à un circuit électrique auquel sont reliées les électrodes, qui effectue les mesures aux bornes des électrodes. Le circuit électronique peut transmettre les résultats de la mesure à un dispositif d'affichage externe au pansement, présent par exemple en permanence dans la même salle que le patient. En variante, le circuit électronique comporte une puce RFID qui est interrogée par le personnel médical lorsque celui-ci est présent aux côtés du patient. Dans ce cas, l'énergie nécessaire au fonctionnement du système de mesure peut être fournie par couplage inductif par le lecteur utilisé par le personnel médical.

Dans le cas où le système de mesure est externe, le pansement peut comporter un identifiant qui est détecté par le système de mesure. Cela permet à ce dernier de sélectionner par exemple des données en mémoire adaptées au pansement identifié, concernant par exemple le système de détection présent et la nature de la structure absorbante, afin de pouvoir en tenir compte pour la mesure.

Le système de mesure peut également être agencé pour vérifier, avant la poursuite des mesures, que la structure absorbante est bien sèche et que le système de détection n'a pas été endommagé. Le cas échéant, des mesures sont effectuées afin de servir de référence pour la suite.

On peut évaluer, grâce à la connaissance de l'évolution du degré de remplissage de la structure absorbante, la quantité d'exsudats reçue par le pansement.

L'invention pourra être mieux comprise à la lecture de la description détaillée qui va suivre, d'exemples non limitatifs de mise en oeuvre de celle-ci, et à l'examen du dessin annexé, sur lequel :
- la figure 1 est une vue schématique, en coupe, d'un exemple de pansement réalisé conformément à l'invention, relié à un système de mesure externe,
- la figure 2 est une vue analogue à la figure 1 d'une variante de réalisation du pansement,
- la figure 3 représente un exemple d'agencement des électrodes au sein d'un système de détection,
- la figure 4 illustre l'utilisation du système de détection de la figure 3 pour déterminer l'étendue de la surface mouillée,
- les figures 5 à 7 sont des vues analogues à la figure 3, de variantes de réalisation du système de détection,
- les figures 8A à 8C sont des oscillogrammes relevés lors de mesures durant un essai,
- les figures 9A et 9B représentent l'évolution de la tension pour différents couples d'électrodes en fonction du degré de remplissage de la structure absorbante, lors de l'essai,
- les figures 10A à 10C illustrent de façon colorimétrique l'évolution du remplissage, lors de l'essai,
- la figure 11 représente un exemple de système de détection utilisé pour l'essai, et
- la figure 12 est un schéma d'un système de mesure utilisé pour l'essai du système de détection de la figure 11.

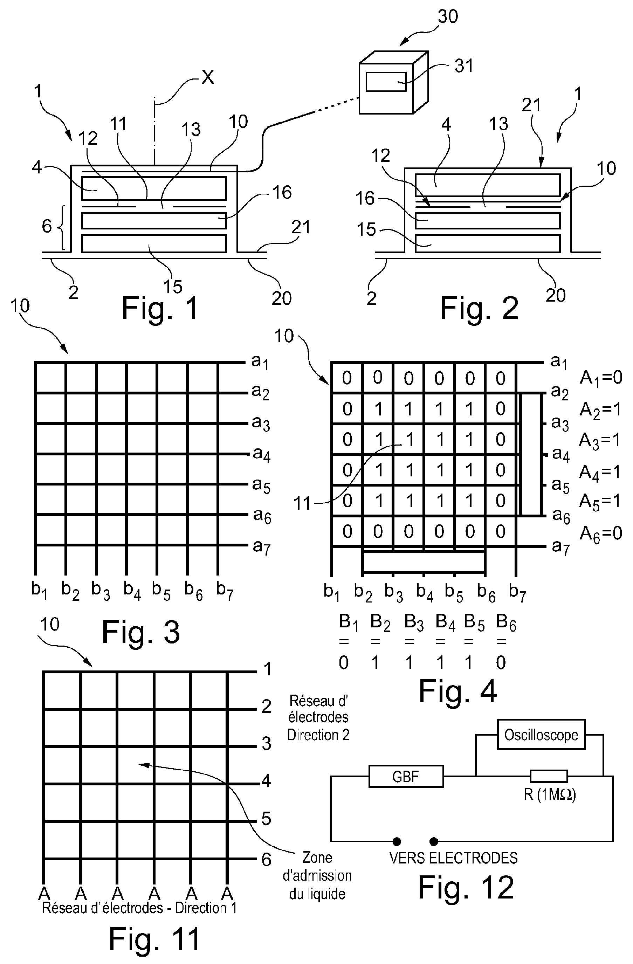

Sur les figures 1 et 2, les proportions réelles des différents éléments constitutifs n'ont pas toujours été respectées, dans un souci de clarté du dessin.

On a représenté à la figure 1 un exemple de pansement 1 selon l'invention, relié à un système de mesure 30 qui comporte par exemple un écran 31 permettant d'afficher des informations liées à l'utilisation du pansement, notamment son taux de remplissage.

Le pansement 1 est conditionné de façon stérile avant la première utilisation.

Dans l'exemple illustré, le pansement 1 est relié par une liaison filaire au système de mesure 30, mais en variante la transmission des informations s'effectue par une liaison sans fil entre le pansement 1 et le système de mesure 30. Dans ce cas, le pansement est équipé d'un circuit électronique qui effectue les mesures et les transmet au système de mesure, lequel peut assurer une partie des calculs ou n'avoir qu'une fonction d'affichage. Le système de mesure peut aussi éventuellement fournir, par couplage inductif, l'énergie nécessaire au fonctionnement du circuit électronique.

Le pansement 1 présente une face 2 d'application sur la plaie et intègre une structure absorbante 4 prévue pour absorber le liquide émis par la plaie.

Le pansement 1 comporte également une structure intermédiaire 6, située entre la face d'application 2 et la structure absorbante 4.

Un système de détection 10 est situé au contact de la structure absorbante 4, par exemple au-dessus de celle-ci, comme illustré à la figure 1.

Le système de détection 10 est sensible à l'étendue de la structure absorbante mouillée par le liquide ayant pénétré dans celle-ci.

De façon à privilégier la diffusion du liquide depuis la face d'application 2 vers une zone d'admission 11 d'étendue restreinte de la structure absorbante, la structure intermédiaire 6 comporte dans l'exemple illustré un film formant barrière 12 aux liquides, disposé au contact de la face inférieure de la structure absorbante 4.

Ce film formant barrière 12 est ajouré en regard de la zone d'admission 11 et présente au moins une ouverture 13 à cet effet. Par ouverture, on entend une zone permettant le passage d'un liquide de part et d'autre du film formant barrière 12. Il peut s'agir d'un trou ou d'une zone poreuse. On comprend que dans ces alternatives, le liquide est apte à passer de part et d'autre du film formant barrière 12.

La structure intermédiaire 6, entre le film 12 formant barrière et la face d'application 2, peut comporter une structure drainante composée d'une couche proximale 15 drainant axialement le liquide, c'est-à-dire sensiblement selon l'axe X perpendiculaire à la plaie, vertical sur la figure 1, et d'une couche distale 16 drainant transversalement le liquide, c'est-à-dire sensiblement perpendiculairement à l'axe X, soit horizontalement sur la figure 1.

La couche proximale 15 est de préférence, comme illustré, adjacente à la face d'application 2. Cette dernière peut être définie par un voile de contact 20 d'un matériau adapté à être en contact avec la plaie, notamment un non tissé.

Le pansement peut comporter une enveloppe externe 21 qui est fixée au voile 20 et qui recouvre les couches 15 et 16, le film 12, la structure absorbante 4 et le système de détection 10. L'enveloppe 21 peut être réalisée dans un matériau semi-perméable, laissant passer l'air.

L'enveloppe 21 peut être fixée au voile 20 à la périphérie du pansement 1, par exemple par soudure ou collage.

La couche drainante proximale 15 peut être réalisée dans un matériau alvéolaire, par exemple une mousse d'un polyuréthane à cellules ouvertes.

La couche drainante distale 16 est par exemple réalisée dans un matériau dit non tissé, notamment de cellulose, et n'être par exemple qu'en cellulose.

Lorsque le pansement 1 est en place sur la plaie, le liquide qui remonte dans la structure intermédiaire 6 pénètre dans la structure absorbante 4 de façon localisée, au travers de l'ajour 13. Ainsi, l'admission du liquide se fait par la ou les zones 11 en regard de l'ajour 13.

Dans l'exemple de la figure 1, le système de détection 10 n'est pas au contact du film 12, étant situé entre la structure absorbante 4 et le dessus de l'enveloppe 21. En variante, le système de détection 10 est situé au contact du film 12, entre ce dernier et la structure absorbante 4, comme illustré à la figure 2. Dans ce cas, le film 12 peut servir de support aux électrodes du système de détection, et par exemple recevoir des pistes conductrices déposées par impression ou métallisation sélective. En cas notamment d'impression des électrodes et de croisement de celles-ci, un isolant peut être déposé localement aux intersections, entre les électrodes.

Dans le cas où le système de détection est couplé à un système de mesure intégré au pansement, une antenne peut également être réalisée sur le support des électrodes. Le système de détection peut être couplé au système de mesure au moyen de connecteurs disposés sur les électrodes, ces derniers permettant une connexion réversible avec le système de mesure.

Le choix du positionnement du système de détection peut se faire, le cas échéant, en fonction de la nature du matériau constituant la structure absorbante, de façon à avoir les meilleurs résultats et/ou la plus grande facilité de fabrication. Ainsi, les deux réseaux d'électrodes peuvent être avantageusement respectivement situés de part et d'autre de la structure absorbante ; ce mode de réalisation permet d'estimer la distribution du liquide à différents endroits dans la structure absorbante.

L'intérêt d'avoir un ajour 13 central constitué d'un ou de plusieurs trous rapprochés est d'avoir une progression concentrique du liquide au sein de la structure absorbante 4 qui s'humidifie, ce qui réduit le risque d'avoir une information faussée sur le taux de remplissage de la structure absorbante.

Le système de détection 10 est réalisé de façon à permettre des mesures en plusieurs emplacements, afin de déterminer l'étendue de la structure absorbante mouillée par le liquide.

Pour réaliser la mesure en plusieurs emplacements, le système de détection peut comporter au moins un réseau d'électrodes, chaque électrode occupant une position connue relativement à la ou aux zones d'admission 11à partir de laquelle ou desquelles la structure absorbante 4 se remplit de liquide, compte tenu de la présence de l'ajour 13.

Ainsi, la distance des électrodes du réseau à chaque zone d'admission 11 varie, de façon régulière ou non. L'écartement des électrodes au sein d'un réseau peut varier et par exemple diminuer lorsque l'on s'éloigne d'une zone d'admission 11, pour bénéficier d'une meilleure précision lorsque le taux de remplissage de la structure absorbante est proche du maximum.

De préférence, le système de détection 10 comporte au moins deux réseaux d'électrodes qui sont réparties dans deux directions différentes du plan selon lequel s'étend la structure absorbante 4, par exemple deux directions perpendiculaires entre elles.

La présence de deux réseaux d'électrodes aᵢ et bⱼ disposés selon une grille est particulièrement avantageuse en ce qu'elle permet d'obtenir un nombre élevé d'emplacements de mesure, mais l'invention n'est pas limitée à une disposition particulière des électrodes, pourvu que l'on puisse obtenir une information sur l'étendue de la structure absorbante 4 mouillée en interrogeant le système de détection 10. Par emplacement de mesure, on entend une zone de la structure absorbante 4 délimitée par des électrodes aᵢ, bⱼ. Dans l'exemple considéré, chaque emplacement de mesure correspond à une maille de la grille.

La figure 3 représente un exemple de système de détection 10 comportant des réseaux d'électrodes aᵢ et bᵢ agencés selon une grille. Sur cette figure, le système de détection comporte un premier réseau d'électrodes aᵢ, ... aₙ et un deuxième réseau d'électrodes b₁ ... bₙ, avec n égal à sept par exemple, comme illustré.

Les électrodes sont des conducteurs électriques qui se croisent sans se toucher mais sont électriquement en contact en plusieurs points de leur longueur avec la structure absorbante 4. Quand la structure absorbante 4 est localement chargée de liquide entre deux électrodes adjacentes aⱼ, aⱼ₊₁, l'impédance entre ces électrodes change et ce changement peut être détecté par une mesure d'une grandeur électrique aux bornes des électrodes. Il en est de même lorsque deux électrodes adjacentes bⱼ, bⱼ₊₁ recouvrent une zone localement chargée de liquide.

A titre d'exemple, sur la figure 4, l'état des mesures effectuées entre différents couples d'électrodes a été représenté, l'ajour 13 étant constitué ici par quatre trous centraux, définissant autant de zones d'admission 11.

L'évaluation du remplissage de la structure absorbante 4 peut consister, comme dans cet exemple, à mesurer la variation de la conductivité électrique entre deux électrodes successives aᵢ ou bⱼ. Dans un exemple, lorsque deux électrodes successives sont en contact électrique en raison de la présence de liquide absorbé par la structure absorbante 4 entre elles, les paramètres Aᵢ ou Bⱼ valent 1. Le taux de remplissage de la structure absorbante peut alors être estimé à partir du produit de la matrice colonne A(A1,A2,A3,A4,A5,A6) par la matrice ligne B(B1,B2,B3,B4,B5,B6).

Un exemple d'estimation du taux de remplissage est présenté à la figure 4, où en raison de la présence de liquide entre les électrodes a₂, a₃, a₄, a₅ et a₆ on obtient la matrice colonne A(A1,A2,A3,A4,A5,A6) qui vaut (0,1,1,1,1,0) et en raison de la présence de liquide entre les électrodes b₂, b₃, b₄, b₅ et b₆ on obtient la matrice ligne B(B1,B2,B3,B4,BS,B6) qui vaut (0,1,1,1,1,0).

Le remplissage de la structure absorbante est évalué dans cet exemple à partir d'une matrice d'humidité C(i,j), déterminée par le produit de la matrice colonne A par la matrice ligne B. Les coefficients de la matrice C(i,j) qui valent 1 représentent chacun une zone de la structure absorbante qui est humide, et ceux qui valent 0 une zone de la structure absorbante qui est encore sèche.

A partir de ces zones mesurées comme humides, le taux de remplissage peut être visualisé de plusieurs manières, à l'aide du système de mesure 30 auquel est relié le système de détection 10.

Le résultat peut être exprimé sous la forme d'une valeur affichée indiquant le taux de remplissage de la structure absorbante 4, par exemple en pourcentage, la valeur 100 % correspondant à un pansement saturé de liquide. On peut encore afficher une cartographie faisant apparaître les zones de la structure absorbante qui sont remplies, parmi l'ensemble des zones de la structure absorbante soumises à détection.

Le système de mesure peut être agencé, le cas échéant, pour effectuer des mesures annexes telles que par exemple indiquer la durée écoulée depuis la pose du pansement, le débit de liquide gagnant la plaie, la durée estimée avant le changement du pansement.

### Essai

On réalise le pansement avec la configuration de la figure 1, en réalisant le voile 20 d'interface de contact avec la plaie avec un non tissé de viscose/polyester, la structure drainante proximale 15 avec une mousse de polyuréthane et la structure drainante distale 16 avec un mélange de polyester/viscose, afin d'avoir une répartition horizontale et homogène des exsudats au niveau de cette structure.

Le film formant barrière 12 est réalisé avec un film de polyuréthane perforé en son centre de 4 trous de diamètre 5 mm. Le film 12 est hydrophobe afin de ne pas favoriser l'étalement du fluide corporel.

La structure absorbante 4 est réalisée en mousse de polyuréthane, ou avec des fibres de cellulose, et peut être avec ou sans superabsorbants tels que des polyacrylates.

L'enveloppe extérieure 21 est par exemple réalisée dans un film de polyuréthane perméable à la vapeur d'eau.

Dans l'essai, le système de détection 10 se compose d'une superposition de deux réseaux d'électrodes perpendiculaires formant une grille, comme illustré à la figure 11. Ces électrodes sont toutes isolées les unes par rapport aux autres, quand le pansement est sec. Les électrodes peuvent être isolées aux croisements par un adhésif de type scotch Kapton^{™} ce qui permet d'obtenir un ensemble d'électrodes autoporteur, c'est-à-dire que le maintien de la position relative des électrodes d'un réseau est assuré par les électrodes de l'autre réseau, et inversement. De plus, le système de détection est de cette façon assez souple, ce qui lui permet de s'adapter facilement à la surface sur laquelle il est appliqué.

L'écart entre les électrodes de chacun des réseaux est par exemple de 15 mm, la largeur d'une électrode est par exemple de 2 mm et la taille de la grille est par exemple de 70 mm par 70 mm.

Le pansement est testé sur un banc de mesure simulant une plaie.

La plaie est matérialisée par un fritté de verre dans lequel on injecte une solution modèle d'exsudat, par exemple une solution aqueuse contenant 0,9 % en masse de NaCl.

Cette solution est injectée à l'aide d'un pousse seringue à un débit de 6 µl/min, ce qui correspond à une valeur de débit proche de celui d'une plaie réelle.

Lors de l'essai, les électrodes de chaque réseau de la grille sont connectées alternativement au système de mesure représenté à la figure 12, qui se compose d'un générateur de fonctions (GBF), d'une résistance R de mesure par exemple de 1 MOhms, et d'un oscilloscope. Il y a ainsi successivement connexion des électrodes A-B, B-C, C-D, DE et E-F pour le réseau associé à la direction 1 et 1-2, 2-3, 3-4, 4-5, 5-6 pour celles du réseau associé à la direction 2.

Le signal sortant du générateur de fonctions est un signal carré ayant une fréquence de 10 kHz et une tension crête de 3V.

Chaque mesure consiste à venir connecter aux bornes du système de mesure un couple d'électrodes successives du système de détection et à relever la tension maximale aux bornes de la résistance R dans ces conditions. Ces connexions successives peuvent être réalisées de façon automatique à l'aide d'interrupteurs électroniques.

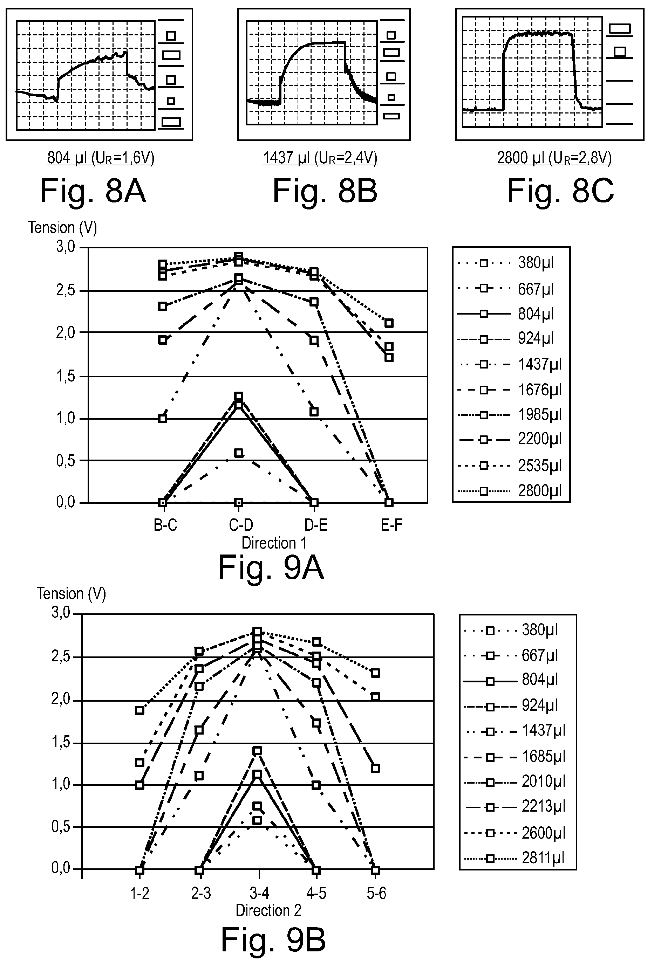

Les figures 8A à 8C représentent, pour 3 volumes délivrés différents, les relevés de la tension aux bornes de la résistance R, lorsque les électrodes 3 et 4 du système de détection de la figure 11 sont connectées. On constate que, lorsque le volume de liquide situé entre les électrodes augmente, la tension aux bornes de la résistance R augmente aussi, de façon progressive. Chaque couple d'électrodes ne joue pas de rôle d'un interrupteur tout ou rien. On remarque aussi que pour les volumes de 804 et 1437 µl, le signal aux bornes de la résistance R n'est pas carré. Ce dernier se rapproche d'un signal carré au fur et à mesure que l'on injecte du liquide entre les électrodes.

Les figures 9A et 9B représentent l'évolution de la tension maximale aux bornes de la résistance R pour chaque couple d'électrodes et pour 5 volumes de liquide délivrés dans le pansement. Pour un couple d'électrodes donné, par exemple le couple C-D du système de détection de la figure 11, la tension aux bornes de la résistance R augmente lorsque la quantité de liquide contenu dans la zone de la structure absorbante située entre ce couple d'électrode croît.

On constate dans l'essai effectué que l'humidification de la structure absorbante se fait manière concentrique, en débutant au centre du pansement pour atteindre l'extérieur de celui-ci.

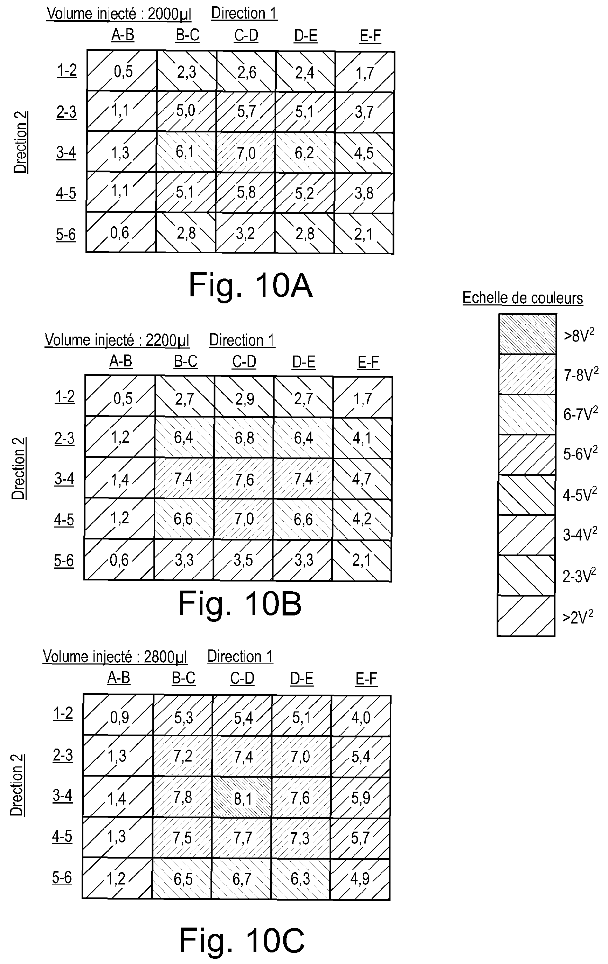

Le taux de remplissage peut être estimé dans l'exemple à partir du produit de la matrice colonne A(A1,A2,A3,A4,A5,A6) par la matrice ligne B(B1,B2,B3,B4,B5,B6). Chaque Aᵢ ou Bⱼ correspond à la valeur de la tension maximale (tension de crête) relevée sur l'oscilloscope pour chaque couple d'électrodes. Les valeurs apparaissant dans les cases coloriées aux figures 10A à 10C représentent le produit de la tension Aᵢ par Bⱼ. Une échelle de couleurs est par exemple utilisée afin de rendre plus visible le remplissage de la structure absorbante (par exemple couleur rouge = très humide, couleur grise = zone peu humide voire sèche).

Cette représentation colorimétrique montre l'humidification concentrique de l'absorbant. Cela confirme la migration verticale et centrale des exsudats provenant de la structure drainante vers la structure absorbante.

D'autre part, on peut noter que pour un volume délivré de 2800 µl, le pansement n'est pas entièrement rempli, car toutes les zones colorées ne sont pas rouges.

L'invention n'est pas limitée à un système de détection 10 présentant une structure particulière.

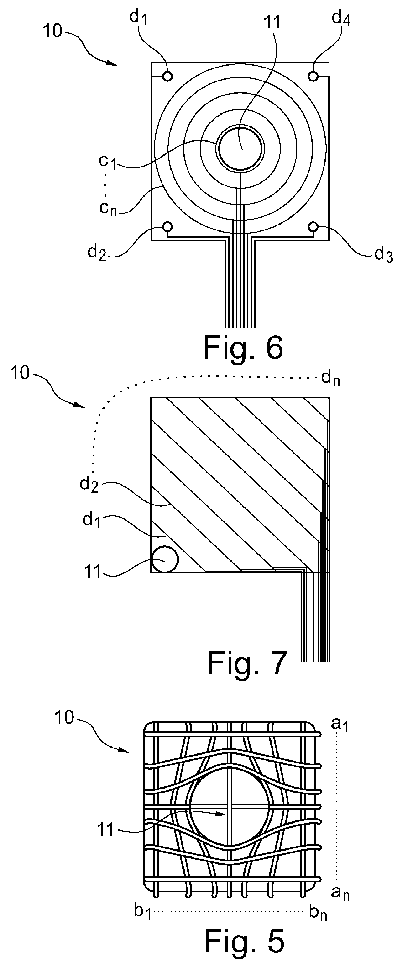

Une variante du système de détection 10 est ainsi illustrée à la figure 5. L'objectif de la géométrie présentée à la figure 5 est d'avoir une meilleure estimation du taux de remplissage lorsque celui-ci est proche de sa valeur maximale. Le principe consiste à écarter les électrodes de la zone d'admission, au centre de la grille, comme si les électrodes devaient contourner un cylindre virtuel placé au centre la grille. Seules les deux électrodes centrales restent rectilignes et l'écart entre deux électrodes successives d'un même réseau d'électrodes est réduit au niveau de l'intersection avec l'électrode de l'autre réseau restée rectiligne.

La variante illustrée à la figure 6 comporte un réseau d'électrodes concentriques c₁, ..., cₙ disposées autour de la zone d'admission 11, centrale, ainsi que des électrodes périphériques d₁, ..., d₄ qui aboutissent dans le voisinage de chacun des coins de la structure absorbante 4. La mesure peut s'effectuer entre chaque couple cⱼ, cⱼ₊ᵢ d'électrodes consécutives, et entre l'électrode cₙ radialement la plus extérieure et chacune des électrodes de coin d₁ à d₄. Les conducteurs d'alimentation des électrodes c₁ à cₙ et d₁ à d₄ peuvent être électriquement isolés de la structure absorbante afin que la mesure s'effectue bien au niveau des électrodes.

La variante illustrée à la figure 7 se caractérise par le fait que le remplissage s'effectue à partir d'un coin, la zone d'admission 11 étant excentrée.

Les électrodes d₁, ..., dₙ sont par exemple disposées, comme illustré, à partir de ce coin en direction du coin opposé, étant par exemple parallèles entre elles et parallèles à la diagonale reliant les deux autres coins. Ainsi, les électrodes sont orientées sensiblement transversalement à la direction dans laquelle se propage le liquide dans la structure absorbante lors du remplissage de celle-ci. La mesure peut s'effectuer entre chaque couple dⱼ,dⱼ₊₁ d'électrodes adjacentes.

L'invention n'est pas limitée aux exemples illustrés. Par exemple, encore d'autres agencements d'électrodes peuvent être utilisés.

L'expression « comportant un » doit se comprendre comme étant synonyme de « comportant au moins un ».

## Revendications

1. Pansement (1) comportant :
- une face d'application (2) sur une plaie ;
- une structure absorbante (4) pour absorber un liquide émis provenant de la plaie ;
- une structure intermédiaire (6) située entre la face d'application (2) et la structure absorbante (4) et agencée pour privilégier la diffusion du liquide depuis la face d'application (2) vers une ou plusieurs zones d'admission (11), d'étendue restreinte, de la structure absorbante (4) ; et
- un système de détection (10) sensible à l'étendue de la structure absorbante mouillée par le liquide ayant pénétré dans celle-ci par la ou les zones d'admission (11).

2. Pansement selon la revendication 1, la structure intermédiaire (6) comportant un film (12) formant barrière au liquide, traversé par une ou plusieurs ouvertures (13) en correspondance avec la ou les zones d'admission (11), notamment entre 1 et 10 ouvertures.

3. Pansement selon la revendication 1 ou 2, la ou les zones d'admission (11) correspondant à moins de 20 % de la surface totale de la structure absorbante (4), en particulier moins de 5 % de la surface totale de la structure absorbante.

4. Pansement selon la revendication 2 ou 3, le film (12) formant barrière étant hydrophobe.

5. Pansement selon l'une quelconque des revendications 2 à 4, le film (12) formant barrière étant en un matériau semi-permeable, par exemple en polyuréthane.

6. Pansement selon l'une quelconque des revendications précédentes, le système de détection (10) comportant un ou plusieurs réseaux d'électrodes, en particulier deux réseaux d'électrodes (a₁,...aₙ; b₁,..., bₙ) agencés sous forme de grille.

7. Pansement selon l'une quelconque des revendications précédentes, le système de détection (10) comportant plusieurs emplacements de mesure répartis sur la structure absorbante, de préférence entre 2 et 100 emplacements de mesure, en particulier de 10 à 50 emplacements de mesure.

8. Pansement selon l'une quelconque des revendications précédentes, le système de détection (10) couvrant une surface de détection qui correspond à au moins 50 % de la surface totale de la structure absorbante.

9. Pansement selon l'une quelconque des revendications précédentes, la structure absorbante (4) étant formée en tout ou partie d'un matériau alvéolaire, notamment à base de mousse de polyuréthane à cellules ouvertes, et/ou d'un matériau fibreux, notamment à base de fibres de cellulose, et comprenant éventuellement un ou plusieurs superabsorbants, notamment des polyacrylates.

10. Pansement selon l'une quelconque des revendications précédentes, comportant entre la face (2) d'application sur la plaie et la structure absorbante (4), une structure (6) drainant le liquide émis provenant de la plaie.

11. Pansement selon la revendication précédente, la structure drainante comportant deux couches superposées, la couche proximale (15) du côté de la plaie drainant axialement le liquide et la couche distale (16) qui lui est superposée drainant transversalement le liquide.

12. Pansement selon la revendication précédente, la couche proximale (15) étant formée en tout ou partie d'un matériau alvéolaire, notamment de mousse de polyuréthane et/ou la couche drainante distale (16) étant formée en tout ou partie d'un matériau non tissé ou d'un tricot, notamment de viscose.

13. Procédé d'estimation du degré de remplissage de la structure absorbante (4) d'un pansement (1) tel que défini dans l'une quelconque des revendications précédentes, dans lequel
(i) on détecte grâce au système de détection (10) l'étendue de la structure absorbante mouillée par le liquide provenant de la plaie ; et
(ii) on détermine en fonction de cette étendue le degré de remplissage de la structure absorbante.

14. Procédé selon la revendication précédente, dans lequel le système de détection (10) est tel que défini selon la revendication 6, et l'étape (i) comprend la mesure d'une grandeur électrique représentative de l'impédance électrique entre deux électrodes du système de détection à l'aide d'un système de mesure (30), en particulier l'étape (i) étant répétée pour mesurer ladite grandeur électrique entre différentes paires d'électrodes successives du ou de chaque réseau d'électrodes.

15. Procédé selon la revendication 13 ou 14, dans lequel on évalue grâce à la connaissance de l'évolution du degré de remplissage la quantité d'exsudats reçue par le pansement.

## Patentansprüche

1. Wundverband (1) mit:
- einer Applikationsfläche (2) zur Auflage auf einer Wunde;
- einer absorbierenden Struktur (4) zum Absorbieren einer von der Wunde abgesonderten Flüssigkeit;
- einer Zwischenstruktur (6), die sich zwischen der Applikationsfläche (2) und der absorbierenden Struktur (4) befindet und dazu ausgebildet ist, die Diffusion der Flüssigkeit von der Applikationsfläche (2) zu einer oder mehreren Einlasszonen von begrenzter Ausdehnung in der absorbierenden Struktur (4) zu bevorzugen; und
- einem Detektionssystem (10), das für die Ausdehnung der absorbierenden Struktur empfindlich ist, die durch die Flüssigkeit angefeuchtet wurde, die in der oder den Einlassronen (11) in sie eingedrungen ist.

2. Wundverband nach Anspruch 1, bei dem die Zwischenstruktur (6) einen Film (12) aufweist, der eine Flüssigkeitsbarriere bildet und von einer oder mehreren Öffnungen durchbrochen ist, die mit der oder den Einlasszonen (11) korrespondieren, insbesondere zwischen 1 und 10 Öffnungen.

3. Wundverband nach Anspruch 1 oder 2 bei dem die Einlasszone (11) oder Einlasszonen weniger als 20% der gesamten Oberfläche der absorbierenden Struktur (4) entsprechen, insbesondere weniger als 5% der gesamten Oberfläche der absorbierenden Struktur.

4. Wundverband nach Anspruch 2 oder 3, bei dem der die Flüssigkeitsbarriere bildende Film (12) hydrophob ist.

5. Wundverband nach einem der Ansprüche 2 bis 4, bei dem der die Flüssigkeitsbarriere bildende Film aus einem semipermeablen Material gebildet ist, insbesondere aus Polyurethan.

6. Wundverband nach einem der vorstehenden Ansprüche, bei dem das Detektionssystem (10) ein oder mehrere Elektrodennetze aufweist, insbesondere zwei Elektrodennetze (a₁, ..., aₙ, b₁, ..., bₙ), die in der Form eines Gitters ausgebildet sind.

7. Wundverband nach einem der vorstehenden Ansprüche, bei dem das Detektionssystem (10) mehrere über die absorbierende Struktur verteilte Messstellen aufweist, vorzugsweise zwischen 2 und 100 Messstellen, insbesondere zwischen 10 und 50 Messstellen.

8. Wundverband nach einem der vorstehenden Ansprüche, bei dem das Detektionssystem (10) eine Detektionsfläche überdeckt, die wenigstens 50% der gesamten Oberfläche der absorbierenden Struktur entspricht.

9. Wundverband nach einem der vorstehenden Ansprüche, bei dem die absorbierende Struktur (4) ganz oder zum Teil aus einem Schaumstoff gebildet ist, insbesondere auf der Basis von offenzelligem Polyurethanschaum, und/oder aus einem Fasermaterial, insbesondere auf der Basis von Zellulosefasern, und ggf. einen oder mehrere Superabsorber aufweist, insbesondere Polyacrylate.

10. Wundverband nach einem der vorstehenden Ansprüche, der zwischen der Wund-Applikationsfläche (2) und der absorbierenden Struktur (4) eine Struktur (6) zur Drainage der von der Wunde abgesonderten Flüssigkeit aufweist.

11. Wundverband nach dem vorstehenden Anspruch, bei dem die Drainagestruktur zwei einander überlagerte Schichten aufweist, wobei die proximale Schicht (15) auf der Seite der Wunde die Flüssigkeit axial drainiert und die distale Schicht (16), die ihr überlagert ist, die Flüssigkeit transversal drainiert.

12. Wundverband nach dem vorstehenden Anspruch, bei dem die proximale Schicht (15) ganz oder zum Teil aus einem Schaumstoff gebildet ist, insbesondere Polyurethanschaum, und/oder die distale Drainageschicht (16) ganz oder zum Teil aus einem V
lies oder Gewirke gebildet ist, insbesondere aus Viskose.

13. Verfahren zur Abschätzung der Füllung der absorbierenden Struktur (4) eines Wundverbandes (1) nach einem der vorstehenden Ansprüche, bei dem
(i) man mit dem Detektionssystem (10) die Ausdehnung der absorbierenden Struktur detektiert, die von der aus der Wunde stammenden Flüssigkeit angefeuchtet wurde; und
(ii) man in Abhängigkeit von dieser Ausdehnung den Füllungsgrad der absorbierenden Struktur bestimmt.

14. Verfahren nach dem vorstehenden Anspruch, bei dem das Detektionssystem gemäß Anspruch 6 ausgebildet ist und der Schritt (i) das Messen einer elektrischen Größe einschließt, die für die elektrische Impedanz zwischen zwei Elektroden des Detektionssystems repräsentativ ist, mit Hilfe eines Messsystems (30), insbesondere wobei der Schritt (i) wiederholt wird, um die genannte elektrische Größe zwischen verschiedenen aufeinanderfolgenden Elektrodenpaaren des oder jedes Elektrodennetzes zu messen.

15. Verfahren nach Anspruch 13 oder 14, bei dem man aufgrund der Kenntnis der Entwicklung des Füllungsgrades die Menge des von dem Wundverband aufgenommenen Exsudats bewertet.

## Claims

1. A dressing (1) comprising:
- a face (2) for application to a wound;
- an absorbent structure (4) for absorbing a liquid discharged from the wound;
- an intermediate structure (6) located between the application face (2) and the absorbent structure (4) and arranged to promote diffusion of the liquid from the application face (2) toward one or more admission zones (11), of restricted extent, of the absorbent structure (4); and
- a detection system (10) sensitive to the extent of the absorbent structure wetted by the liquid having penetrated into said structure via the admission zone(s) (11).

2. The dressing as claimed in claim 1, the intermediate structure (6) comprising a film (12) forming a barrier to the liquid, which film is pierced with one or more openings (13) in correspondence with the admission zone(s) (11), especially between 1 and 10 openings.

3. The dressing as claimed in claim 1 or 2, the admission zone(s) (11) corresponding to less than 20% of the total area of the absorbent structure (4), in particular less than 5% of the total area of the absorbent structure.

4. The dressing as claimed in claim 2 or 3, the film (12) forming a barrier being hydrophobic.

5. The dressing as claimed in any one of claims 2 to 4, the film (12) forming a barrier being made of a semi-permeable material, for example of polyurethane.

6. The dressing as claimed in any one of the preceding claims, the detection system (10) comprising one or more networks of electrodes, in particular two networks of electrodes (a₁,...aₙ; b₁, ..., bₙ) arranged in the form of a grid.

7. The dressing as claimed in any one of the preceding claims, the detection system (10) comprising a plurality of measuring locations distributed over the absorbent structure, preferably between 2 and 100 measurement locations, in particular from 10 to 50 measurement locations.

8. The dressing as claimed in any one of the preceding claims, the detection system (10) covering a detection area that corresponds to at least 50% of the total area of the absorbent structure.

9. The dressing as claimed in any one of the preceding claims, the absorbent structure (4) being entirely or partially formed from a cellular material, especially based on an open cell polyurethane foam, and/or from a fibrous material, especially based on cellulose fibers, and optionally comprising one or more superabsorbents, especially polyacrylates.

10. The dressing as claimed in any one of the preceding claims, comprising, between the face (2) for application to the wound and the absorbent structure (4), a structure (6) draining the liquid discharged from the wound.

11. The dressing as claimed in the preceding claim, the draining structure comprising two superposed layers, the proximal layer (15) on the side of the wound draining the liquid axially and the distal layer (16) which is superposed thereon draining the liquid transversely.

12. The dressing as claimed in the preceding claim, the proximal layer (15) being entirely or partially formed from a cellular material, especially from polyurethane foam and/or the distal draining layer (16) being entirely or partially formed from a nonwoven material or a knit, especially from viscose rayon.

13. A method for estimating the degree of saturation of the absorbent structure (4) of a dressing (1) such as defined in any one of the preceding claims, in which
(i) by virtue of the detection system (10) the extent of the absorbent structure wetted by the liquid discharged from the wound is detected; and
(ii) depending on this extent the degree of saturation of the absorbent structure is determined.

14. The method as claimed in the preceding claim, in which the detection system (10) is such as defined in claim 6, and step (i) comprises measuring an electrical quantity representative of the electrical impedance between two electrodes of the detection system using a measurement system (30), in particular step (i) being repeated in order to measure said electrical quantity between various successive pairs of electrodes of the or each network of electrodes.

15. The method as claimed in claim 13 or 14, in which, by virtue of knowledge of the course of the degree of saturation, the amount of exudates received by the dressing is evaluated.
